# EUROPÄISCHE PATENTSCHRIFT

(11) **EP 2 022 534 B1**
(45) Veröffentlichungstag und Bekanntmachung des Hinweises auf die Patenterteilung: **29.09.2010**
(21) Anmeldenummer: 08104711.0
(22) Anmeldetag: 11.07.2008
(51) Int. Cl.: A61N 5/10

(54) **Steuervorrichtung zur Steuerung eines Bestrahlungsvorgangs, Partikeltherapieanlage sowie Verfahren zur Bestrahlung eines Zielvolumens**
Control device for controlling an irradiation procedure, particle therapy facility and method for irradiating a target volume
Dispositif de commande destiné à commander un processus de rayonnement, installation de thérapie à particules ainsi que procédé de rayonnement d'un volume cible

(30) Priorität: 01.08.2007 DE 102007036035
(43) Veröffentlichungstag der Anmeldung: 11.02.2009
(73) Patentinhaber: Siemens Aktiengesellschaft, 80333 München (DE)
(72) Erfinder: Kaiser, Werner, 91052 Erlangen (DE); van Haßelt, Peter, 91058 Erlangen (DE)

(56) Entgegenhaltungen:
- EP-A- 1 477 206
- EP-A- 1 733 757
- WO-A-00/40064
- WO-A-2005/120641
- WO-A-2006/082650
- US-A- 5 039 867
- US-A- 6 034 377
- US-B1- 6 891 177
- HABERER T ET AL: "MAGNETIC SCANNING SYSTEM FOR HEAVY ION THERAPY" NUCLEAR INSTRUMENTS & METHODS IN PHYSICS RESEARCH, SECTION - A:ACCELERATORS, SPECTROMETERS, DETECTORS AND ASSOCIATED EQUIPMENT, ELSEVIER, AMSTERDAM, NL, Bd. A330, Nr. 1/02, 10. Juni 1993 (1993-06-10), Seiten 296-305, XP000385496 ISSN: 0168-9002

## Beschreibung

Die Erfindung betrifft eine Steuervorrichtung zur Steuerung eines Bestrahlungsvorgangs bei einer Partikeltherapieanlage, eine Partikeltherapieanlage sowie ein Verfahren zur Bestrahlung eines Zielvolumens.

Die Partikeltherapie ist ein etabliertes Verfahren zur Behandlung von Gewebe, insbesondere von Tumorerkrankungen. Bestrahlungsverfahren, wie sie in der Partikeltherapie eingesetzt werden, können jedoch auch in nicht-therapeutischen Gebieten Anwendung finden kann. Hierzu gehören beispielsweise Forschungsarbeiten im Rahmen der Partikeltherapie, die an nicht-lebenden Phantomen oder Körpern durchgeführt werden, Bestrahlungen von Materialien, etc. Hierbei werden üblicherweise geladene Partikel auf hohe Energien beschleunigt, zu einem Partikelstrahl geformt und auf ein zu bestrahlendes Objekt gerichtet. Der Partikelstrahl dringt in das Objekt ein und gibt dort an einem definierten Ort seine Energie ab, was zu einer Zerstörung des sich dort befindlichen Gewebes führt. Als Partikel kommen üblicherweise Protonen, Kohlenstoffionen aber auch Pionen, Heliumionen oder andere Ionensorten zum Einsatz.

Im Vergleich zu Bestrahlungsverfahren mit Röntgenstrahlen zeichnet sich die Partikeltherapie dadurch aus, dass die Partikel des Partikelstrahls den Hauptteil ihrer Energie in einem relativ gut abzugrenzenden Bereich innerhalb des Zielvolumens abgeben. Wo genau der Partikelstrahl mit dem Zielvolumen wechselwirkt, d.h. in welcher Tiefe des Zielvolumens bezogen auf die Strahlrichtung, hängt vorwiegend von der Energie des Partikelstrahls ab. Je höher die Energie des Partikelstrahls ist, desto weiter dringt der Partikelstrahl in das Zielvolumen ein und desto tiefer liegt der Bereich, in dem die Partikel den Hauptteil ihrer Energie an das Zielvolumen abgeben. Diese Eigenschaft der Partikeltherapie erlaubt es, ein zu bestrahlendes Zielvolumen innerhalb eines Objektes relativ genau zu bestrahlen und umliegende Bereiche zu schonen.

Andererseits bedeutet dies auch, dass ein Zielvolumen oftmals in mehreren Schichten bestrahlt werden muss. Dies ist insbesondere dann der Fall, wenn das Zielvolumen in Strahlrichtung derart groß ist, dass die Schicht, in der Partikel mit einer definierten Energie ihre Energie abgeben, so dünn ist, dass damit das Zielvolumen nicht durch den Partikelstrahl mit Partikeln der definierten Energie erfasst werden kann. In diesem Fall erfolgt oftmals eine schichtweise Bestrahlung des Zielvolumens, wobei bei jeder Schicht die Energie der Partikel an die Tiefe dieser Schicht in dem zu bestrahlenden Objekt angepasst wird.

Die Energie der Partikel wird ihrerseits oftmals nicht nur unmittelbar vor dem zu bestrahlenden Objekt eingestellt, sondern weit vorher im Strahlverlauf, beispielsweise im Bereich des Beschleunigers, mit dem Partikel auf eine zur Bestrahlung notwendige Energie beschleunigt werden, oder im Bereich des Hochenergiestrahltransportsystems, mit dem Partikel von einem Beschleuniger zu einem Bestrahlungsraum transportiert werden.

Besonders bei komplexen Bestrahlungsvorgängen, bei denen mehrere Schichten gegebenenfalls mehrmals bestrahlt werden, erfordert dies eine entsprechend komplexe Steuerung der Partikeltherapieanlage und Abstimmung der Energie des Partikelstrahls auf die zu bestrahlenden Schichten.

Die EP-A-1 733 757 offenbart ein Bestrahlungsverfahren, bei dem die Breite eines Bragg-Peaks mit Hilfe eines sogenannten Range Modulator Wheel variabel eingestellt werden kann.

Die WO 2006/082650 offenbart ein Bestrahlungsverfahren, bei dem hintereinander angeordnete Schichten in vordefinierter

Weise bestrahlt können, wobei eine Schicht auch mehrfach bestrahlt werden kann.

Die EP-A-1 477 206 offenbart ein Bestrahlungsverfahren, bei dem Rasterpunkte in einer Schicht mehrfach bestrahlt werden können.

Es ist daher die Aufgabe der Erfindung, eine Steuervorrichtung zur Steuerung eines Bestrahlungsvorgangs bei einer Partikeltherapieanlage sowie eine Partikeltherapieanlage anzugeben, mit denen auch bei komplexen Bestrahlungsvorgängen mit einer komplexen Abstimmung der Energie des Partikelstrahls eine günstige und geringe Belastung der Komponenten einer Partikeltherapieanlage erreicht wird. Weiterhin ist es die Aufgabe der Erfindung ein Verfahren zur Bestrahlung eines Zielvolumens anzugeben, das auch bei komplexen Bestrahlungsvorgängen mit einer komplexen Abstimmung der Energie des Partikelstrahls Komponenten einer Partikeltherapieanlage in lediglich geringem Umfang belastet.

Die Erfindung wird demnach gelöst durch eine Partikeltherapieanlage nach Anspruch 1 oder 13, sowie durch ein Bestrahlungsverfahren nach Anspruch 7 oder 14. Das Bestrahlungsverfahren wird an nicht-lebenden Körpern, Materialien und/oder Phantomen durchgeführt. Vorteilhafte Weiterbildungen finden sich in den Merkmalen der abhängigen Ansprüche.

Die Steuervorrichtung zur Steuerung eines Bestrahlungsvorgangs bei einer Partikeltherapieanlage ist dabei derart ausgebildet, dass ein Zielvolumen durch zumindest zwei Bestrahlungsvorgänge bestrahlt wird, wobei bei jedem Bestrahlungsvorgang eine Energie eines Partikelstrahls derart variiert wird, dass das Zielvolumen schichtweise in Schichten, die in Strahlrichtung räumlich hintereinander angeordnet sind, bestrahlt wird. Eine Reihenfolge, in der die Schichten des Zielvolumens bei einem der Bestrahlungsvorgänge bestrahlt werden, wird - bezogen auf eine Einfallsrichtung des Partikelstrahls - von Bestrahlungsvorgang zu Bestrahlungsvorgang variiert.

Als Bestrahlungsvorgang wird dabei ein Teil einer Bestrahlungssitzung verstanden, bei dem zumindest ein Teil der Schichten des Zielvolumens sukzessive nacheinander bestrahlt werden. Die Bestrahlung der Schichten erfolgt während eines Bestrahlungsvorgangs in einer Richtung (bezogen auf die Strahlrichtung), d.h. entweder in Strahlrichtung oder gegen Strahlrichtung.

Es wurde dabei erkannt, dass - falls Schichten eines Zielvolumens, die in mehreren Bestrahlungsvorgängen stets in einer gleichen Reihenfolge bestrahlt werden - die Energie des Partikelstrahls entsprechend eingestellt werden muss. Dieses Einstellen der Energie führt zu einer ungünstigen Belastung von Komponenten einer Partikeltherapieanlage, insbesondere bei dem Übergang von einem Bestrahlungsvorgang zum nächsten Bestrahlungsvorgang, vor allem, wenn die Energieänderung große Sprünge aufweist. Wenn beispielsweise Schichten eines Zielvolumens stets in einer Richtung - z.B. stets von vorn nach hinten bezogen auf die Strahlrichtung - bestrahlt werden, muss die Energie bei dem Übergang von einem Bestrahlungsvorgang zu dem nächsten Bestrahlungsvorgang von einem maximalen Wert zu einem minimalen Wert geregelt werden. Dieser abrupte Sprung in der Energie des Partikelstrahls führt zu einer ungünstigen Belastung der Komponenten einer Partikeltherapieanlage.

Insbesondere, wenn bei einer Partikeltherapieanlage supraleitende Magnete zur Strahlführung eingesetzt werden, muss ein Magnetfeld dieser supraleitenden Magnete der Energie des Partikelstrahls angepasst werden. Wenn nun die Energie des Partikelstrahls sich abrupt ändert, führt die schnelle Anpassung des Magnetfeldes zu Wechselfeldverlusten (im Folgenden auch als "AC-Verluste" genannt). Da AC-Verluste zu erhöhtem Kühlleistungsbedarf führen, werden insgesamt Komponenten der Partikeltherapieanlage stark belastet. Bisher wurde das Problem dadurch gelöst, dass entweder eine erhöhte Kühlleistung bereitgestellt wurde oder dass die Änderung des Magnetfeldes nur langsam durchgeführt wird. Beides ist mit Nachteilen verbunden, entweder mit erhöhten Kosten oder mit einem erhöhten Zeitaufwand bei komplexen Bestrahlungsvorgängen. Eine starke Belastung von Komponenten einer Partikeltherapieanlage ist jedoch nicht nur auf supraleitende Magnete beschränkt, auch wenn hier die Belastung aufgrund der deutlich erhöhten Kühlleistung stärker erkennbar wird.

Die Steuervorrichtung erlaubt es, die Reihenfolge, in der die Schichten bei einem Bestrahlungsvorgang bestrahlt werden, von Bestrahlungsvorgang zu Bestrahlungsvorgang zu variieren. Hierdurch wird es ermöglicht, große Übergänge bzw. Sprünge der Energie des Partikelstrahls zu vermeiden.

Die Reihenfolge, in der die Schichten bei einem Bestrahlungsvorgang bestrahlt werden, wird vorzugsweise so geändert, dass beim Übergang von Bestrahlungsvorgang zu Bestrahlungsvorgang die gleiche Schicht oder benachbarte Schichten bestrahlt werden, insbesondere direkt benachbarte Schichten.

Die Energie des Partikelstrahls wird hierdurch in lediglich geringem Umfang geändert, von Schicht zu Schicht und von Bestrahlungsvorgang zu Bestrahlungsvorgang. Die Reihenfolge der Schichten, die bei einem Bestrahlungsvorgang bestrahlt werden, kann bei dem nächsten Bestrahlungsvorgang beispielsweise umgedreht werden.

Die Steuervorrichtung ist derart ausgebildet, dass die Reihenfolge der Schichten, die bei einem Bestrahlungsvorgang bestrahlt werden, von Bestrahlungsvorgang zu Bestrahlungsvorgang bezogen auf die Einfallsrichtung alternierend durchlaufen wird. Wenn bei einem ersten Bestrahlungsvorgang die Schichten beispielsweise von vorne nach hinten bezogen auf die Strahlrichtung bestrahlt werden, wird diese Richtung im nächsten Bestrahlungsvorgang umgedreht, so dass die Schichten nun von hinten nach vorn bestrahlt werden. Die Energie des Partikelstrahls ändert sich hierdurch von einem minimalen zu einem maximalen Wert und anschließend von dem maximalen Wert wieder hin zum minimalen Wert. Die so entstehende Belastung für Komponenten der Partikeltherapieanlage ist vergleichsweise gering.

Die Bestrahlung einer der Schichten kann dabei durch ein Scattering-Verfahren oder durch ein Scan-Verfahren erfolgen. Bei dem Scattering-Verfahren wird der Partikelstrahl in seiner lateralen Ausdehnung aufgeweitet und an die Dimensionen der zu bestrahlenden Schicht angepasst, beispielsweise durch einen Kollimator. Bei dem Scan-Verfahren hingegen behält der Partikelstrahl eine vergleichsweise geringe laterale Ausdehnung, z.B. von wenigen Millimetern, und wird sukzessive auf verschiedene Punkte der Schicht gelenkt, so dass die Schicht durch den Partikelstrahl z.B. rasterförmig "gescannt" wird.

Bei den zumindest zwei Bestrahlungsvorgängen können stets die gleichen Schichten im Zielvolumen bestrahlt werden, oder aber auch unterschiedliche Schichten im Zielvolumen bestrahlt werden. So können beispielsweise die Schichten im Zielvolumen von Bestrahlungsvorgang zu Bestrahlungsvorgang neu positioniert werden, wenn beispielsweise bei jedem Bestrahlungsvorgang ein unterschiedliches Bestrahlungsfeld bestrahlt wird.

Die Einfallsrichtung des Partikelstrahls kann dabei von Bestrahlungsvorgang zu Bestrahlungsvorgang gleich bleiben oder aber auch variiert werden.

Die Steuervorrichtung muss dabei nicht zwangsläufig eine abgeschlossene Einheit einer Partikeltherapieanlage sein. Die Steuervorrichtung kann genauso gut auf einzelne Teilvorrichtungen aufgeteilt sein, die in ihrem Zusammenwirken die Funktionalität der Steuervorrichtung bereitstellen. Beispielsweise kann die Steuervorrichtung durch eine Steuervorrichtung zur Steuerung der Energie des Partikelstrahls, und durch eine Steuervorrichtung zur Umsetzung von in einem Therapieplan hinterlegten Steuerkommandos realisiert sein, die derart ausgebildet ist, dass eine Reihenfolge von zu bestrahlenden Schichten von Bestrahlung zu Bestrahlungsvorgang variiert wird.

Die erfindungsgemäße Partikeltherapieanlage umfasst:
- eine Partikelquelle zur Erzeugung von Partikeln,
- einen Beschleuniger zur Beschleunigung der Partikel und zur Bereitstellung eines hochenergetischen Partikelstrahls,
- ein Hochenergiestrahltransportsystem zur Führung eines aus den beschleunigten Partikeln geformten Partikelstrahls in einen Bestrahlungsraum,
- eine Energieeinstellvorrichtung, mit der eine Energie des Partikelstrahls einstellbar ist, und
- eine Steuervorrichtung zur Steuerung eines Bestrahlungsvorgangs wie vorstehend beschrieben, mit der die Energieeinstellvorrichtung steuerbar ist.

Die Energieeinstellvorrichtung kann beispielsweise den Beschleuniger derart steuern, dass die Partikel derart beschleunigt werden, dass eine gewünschte Energie des Partikelstrahls erreicht wird. Eine solche Einstellung der Energie des Partikelstrahls ist insbesondere bei einem Beschleuniger, der als Synchrotron ausgebildet ist, möglich. Bei einem Beschleuniger, der als Zyklotron ausgebildet ist, kann eine Einstellung der Energie des Partikelstrahls z.B. mithilfe eines Energie-Selektions-Systems nach Beschleunigung der Partikel erfolgen.

Insbesondere kann der Beschleuniger oder das Hochenergiestrahltransportsystem zumindest einen supraleitenden Magneten umfassen. In diesem Fall wirkt sich die Steuervorrichtung zur Steuerung eines Bestrahlungsvorgangs besonders vorteilhaft aus, da - wie bereits beschrieben - der supraleitende Magnet nur geringe AC-Verluste erzeugen wird, auch wenn komplexe Bestrahlungsvorgänge ausgeführt werden. Die Energieeinstellvorrichtung steuert dabei die Energie des Partikelstrahls in Strahlrichtung insbesondere vor dem supraleitenden Magneten, d.h. bevor der Partikelstrahl durch das Magnetfeld des supraleitenden Magneten geführt wird. Dessen Magnetfeld ist wiederum auf die Energie des Partikelstrahls abgestimmt. Dies kann beispielsweise dadurch erfolgen, dass die Steuervorrichtung zur Steuerung des Bestrahlungsvorgangs, mit dem die Energie des Partikelstrahls eingestellt wird, gleichzeitig das Magnetfeld während eines Bestrahlungsvorgangs steuert.

Bei dem erfindungsgemäßen Verfahren zum Steuern der Energie eines Partikelstrahls während einer Bestrahlung eines Zielvolumens, bei der ein Zielvolumen durch zumindest zwei Bestrahlungsvorgänge bestrahlt wird, wird die Energie eines Partikelstrahls derart variiert,
- dass bei jedem Bestrahlungsvorgang das Zielvolumen schichtweise in Schichten, die in Strahlrichtung räumlich hintereinander angeordnet sind, bestrahlt wird, und
- dass eine Reihenfolge, in der die Schichten des Zielvolumens bei einem der Bestrahlungsvorgänge bestrahlt werden, - bezogen auf eine Einfallsrichtung des Partikelstrahls - von Bestrahlungsvorgang zu Bestrahlungsvorgang variiert wird.

Die Reihenfolge der Schichten wird von Bestrahlungsvorgang zu Bestrahlungsvorgang alternierend durchlaufen, jeweils bezogen auf die Einfallsrichtung des Partikelstrahls. Die Bestrahlung wird an nicht-lebenden Körpern, Materialien und/oder Phantomen durchgeführt.

Die Energie des Partikelstrahls kann eingestellt werden, bevor der Partikelstrahl durch einen supraleitenden Magneten eines Beschleunigers oder eines Hochenergiestrahltransportsystems geführt wird. In diesem Fall wird das Magnetfeld des supraleitenden Magneten an die Energie des Partikelstrahls angepasst.

Ausführungsformen der Erfindung sowie Weiterbildungen gemäß den Merkmalen der unabhängigen Ansprüche werden anhand der folgenden Zeichnung näher erläutert, ohne jedoch darauf beschränkt zu sein. Es zeigen:
- Fig. 1: einen schematischen Aufbau einer Partikeltherapieanla- ge,
- Fig. 2: und Fig. 3 die schichtweise Bestrahlung eines Zielvo- lumens bei zwei aufeinander folgenden Bestrahlungsvor- gängen, wobei dieselben Schichten bestrahlt werden und ein Partikelstrahl mit derselben Einfallsrichtung an- gewendet wird, und
- Fig. 4: und Fig. 5 die schichtweise Bestrahlung eines Zielvo- lumens während zwei aufeinander folgenden Bestrah- lungsvorgänge, wobei jeweils unterschiedliche Schich- ten bestrahlt werden mit einem Partikelstrahl mit ge- änderter Einfallsrichtung.

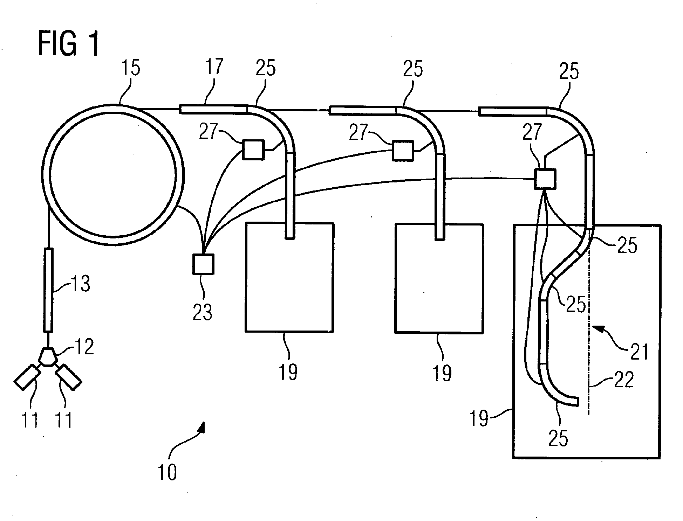

Figur 1 zeigt einen schematischen Überblick über den Aufbau einer Partikeltherapieanlage 10. In einer Partikeltherapieanlage 10 erfolgt insbesondere eine Bestrahlung eines Körpers, insbesondere eines tumorerkrankten Gewebes, mit einem Partikelstrahl.

Als Partikel werden vornehmlich Ionen wie beispielsweise Protonen, Pionen, Heliumionen, Kohlenstoffionen oder andere Ionensorten eingesetzt. Üblicherweise werden derartige Partikel in einer Partikelquelle 11 erzeugt. Wenn, wie in Figur 1 dargestellt, zwei Partikelquellen 11 vorhanden sind, die zwei verschiedene Ionensorten erzeugen, kann zwischen diesen beiden Ionensorten innerhalb eines kurzen Zeitintervalls umgeschaltet werden. Dazu wird beispielsweise ein Schaltmagnet 12 verwendet, der zwischen den Ionenquellen 11 einerseits und einem Vorbeschleuniger 13 andererseits angeordnet ist. Z.B. kann hierdurch die Partikeltherapieanlage 10 mit Protonen und mit Kohlenstoffionen gleichzeitig betrieben werden.

Die von der oder einer der Ionenquellen 11 erzeugten und gegebenenfalls mit dem Schaltmagneten 12 ausgewählten Ionen werden in dem Vorbeschleuniger 13 auf ein erstes Energieniveau beschleunigt. Der Vorbeschleuniger 13 ist beispielsweise ein Linearbeschleuniger (LINAC für engl.: "LINear ACcelerator"). Anschließend werden die Partikel in einen Beschleuniger 15, beispielsweise ein Synchrotron oder Zyklotron, eingespeist. In dem Beschleuniger 15 werden sie auf hohe Energien, wie sie zur Bestrahlung nötig sind, beschleunigt. Nachdem die Partikel den Beschleuniger 15 verlassen, führt ein Hochenergiestrahltransportsystem 17 den Partikelstrahl zu einem oder mehreren Bestrahlungsräumen 19. In einem Bestrahlungsraum 19 werden die beschleunigten Partikel auf einen zu bestrahlenden Körper gerichtet. Je nach Ausgestaltung erfolgt dies von einer festen Richtung (in so genannten "fixed beam"-Räumen) aus oder aber über eine um eine Achse 22 bewegliche rotierbare Gantry 21 von verschiedenen Richtungen aus.

Die Energie des Partikelstrahls kann bei der hier gezeigten Partikeltherapieanlage beispielsweise dadurch eingestellt werden, dass der Beschleuniger 15 entsprechend angesteuert wird. Eine Feineinstellung der Energie des Partikelstrahls kann gegebenenfalls noch im Behandlungsraum 19 stattfinden, insbesondere unmittelbar bevor der Partikelstrahl auf ein zu bestrahlendes Objekt trifft.

Die Energie des Partikelstrahls wird durch eine Steuervorrichtung 23 gesteuert, die die entsprechenden Komponenten, mit der die Energie des Partikelstrahls eingestellt wird, ansteuert. Beispielsweise kann die Steuervorrichtung 23 den Beschleuniger 15 ansteuern, so dass die Partikel auf jeweils eine gewünschte Energie beschleunigt werden. Mit der Steuervorrichtung 23 wird folglich die Energie des Partikelstrahls variiert, so dass bei komplexen Bestrahlungsvorgängen eine Bestrahlung schichtweise erfolgen kann, in dem über die Energie des Partikelstrahls die zu bestrahlende Schicht festgelegt wird. Die Steuervorrichtung 23 ist dabei derart ausgebildet, dass eine Reihenfolge, in der die Schichten des Zielvolumens bestrahlt werden, von Bestrahlungsvorgang zu Bestrahlungsvorgang variiert wird.

Zur Beschleunigung, zur Formung und zur Umlenkung des Partikelstrahls wird in einer Partikeltherapieanlage eine Vielzahl von Magnetkomponenten 25 eingesetzt. Einige dieser Magnetkomponenten 25, insbesondere Dipolmagnete, sind in Fig. 1 dargestellt. Zumindest einer diese Dipolmagnete kann insbesondere supraleitend ausgebildet sein, beispielsweise ein Dipolmagnet der Gantry oder ein Dipolmagnet des Hochenergiestrahltransportsystems.

Die Steuervorrichtung 23 steuert dabei ebenso die Magnetkomponenten 25, durch die der Partikelstrahl geführt wird, so dass das Magnetfeld der Magnetkomponenten 25, das für eine korrekte Strahlführung erforderlich ist, auf die Energie des Partikelstrahls abgestimmt wird. Diese Steuerung kann direkt oder auch indirekt erfolgen, indem die Steuervorrichtung 23 entsprechende Steuerbefehle an weitere Steuervorrichtungen 27 für die entsprechenden Magnetkomponenten sendet. Insbesondere bei supraleitenden Magnetkomponenten, wie beispielsweise supraleitende Dipolmagnete, geht eine derartige Anpassung des Magnetfeldes stets mit einem so genannten AC-Verlust einher. Dieser AC-Verlust ist umso größer, je schneller und stärker eine derartige Anpassung erfolgt.

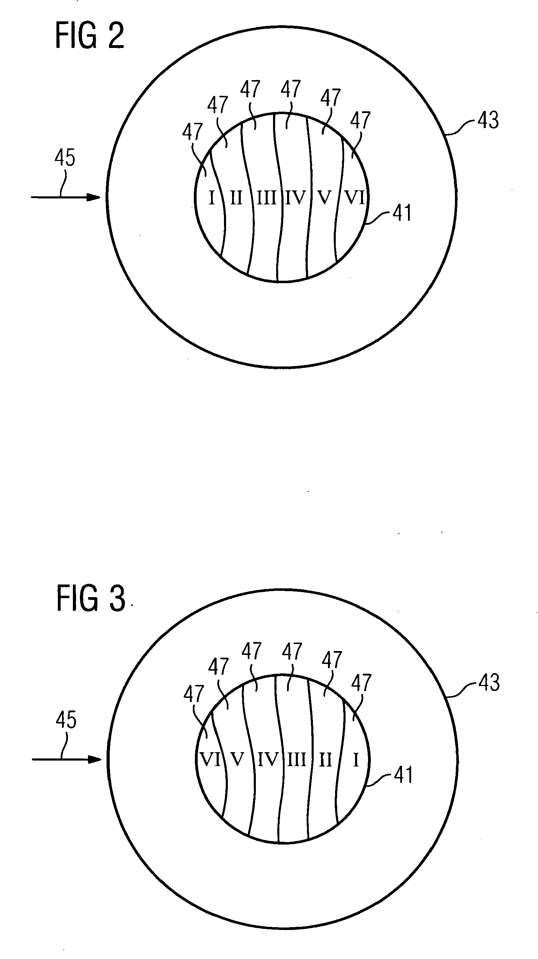

Um diesen Effekt zu verringern, ist die Steuervorrichtung 23 derart ausgebildet, dass eine Bestrahlung eines Zielvolumens wie anhand von Fig. 2 bis Fig. 5 nachfolgend näher erläutert erfolgen kann.

Der anhand der Figur 1 dargestellte Grundaufbau einer Partikeltherapieanlage 10 ist typisch für viele Partikeltherapieanlagen, kann aber auch hiervon abweichen. Beispielsweise kann eine Partikeltherapieanlage lediglich einen Raum zur Bestrahlung aufweisen. Falls ein Zyklotron zur Beschleunigung von Partikeln eingesetzt wird, kann zusätzlich ein Energie-Selektions-System im Hochenergiestrahltransportsystem angeordnet werden, mit dem die Energie des Partikelstrahls eingestellt werden kann.

Fig. 2 zeigt schematisch ein kugelförmiges Zielvolumen 41, das sich in einem zu bestrahlenden Objekt 43 befindet. Der Pfeil 45 vor dem Objekt 43 zeigt die Strahlrichtung des Partikelstrahls. Eine Einstellung der räumlichen Beziehung zwischen Strahlrichtung des Partikelstrahls und dem zu bestrahlenden Objekt kann beispielsweise über eine Führung des Partikelstrahls mithilfe einer beweglichen Gantry erfolgen. Alternativ und/oder zusätzlich kann die Einstellung auch durch Positionierung des Objekts in Bezug auf den Partikelstrahl erfolgen, beispielsweise mit einer als Roboterarm ausgebildeten Positioniervorrichtung.

Das zu bestrahlende Zielvolumen 41 weist dabei eine Dimension in Strahlrichtung auf, so dass das Zielvolumen 41 nicht durch einen Partikelstrahl mit einer definierten Energie vollständig erfasst wird. Um das Zielvolumen 41 dennoch gänzlich zu bestrahlen, wird die Energie des Partikelstrahls sukzessive variiert, so dass jeweils eine Schicht 47 des Zielvolumens 41 bestrahlt wird. Die Form der Schichten 47 kann dabei von einer parallelen Anordnung abweichen. Dies kann beispielsweise durch eine inhomogene Gewebsverteilung innerhalb des zu bestrahlenden Objekts 43 bzw. innerhalb des Zielvolumens 41 bedingt sein. Eine Anpassung der lateralen Ausdehnung des Partikelstrahls an die jeweils zu bestrahlende Schicht 47 kann auf verschiedene Weise erfolgen. Beim Scattering-Verfahren beispielsweise kann ein aufgeweiteter Partikelstrahl mittels Kollimatoren an die Ausdehnung der jeweils zu bestrahlenden Schicht 47 angepasst werden. Beim Scan-Verfahren hingegen kann ein nadelförmiger Partikelstrahl sukzessive auf verschiedene Punkte innerhalb der zu bestrahlenden Schicht 47 gerichtet werden, solange, bis eine Bestrahlung der gesamten Schicht 47 erfolgt ist.

Während eines Bestrahlungsvorgangs wird zumindest ein Teil der Schichten 47, vorzugsweise alle Schichten 47, in einer Richtung - bezogen auf die Einfallsrichtung des Partikelstrahls - bestrahlt. Die römischen Ziffern I bis VI innerhalb der Schichten 47 kennzeichnen dabei die Reihenfolge, in der die Schichten während des anhand von Fig. 2 dargestellten Bestrahlungsvorgangs bestrahlt werden. Zuerst wird die in Strahlrichtung vorne liegende Schicht 47 mit der römischen Ziffer I bestrahlt. Anschließend werden sukzessive immer tiefer liegende Schichten bestrahlt, bis zuletzt die in Strahlrichtung hinten liegende Schicht 47 mit der römischen Ziffer VI bestrahlt wird.

Anhand der von Fig. 3 ist die Reihenfolge der Schichten 47 dargestellt, wie sie bei dem darauf folgenden Bestrahlungsvorgang bestrahlt werden. Die Reihenfolge der Schichten 47 ist nun variiert, und zwar derart, dass bei dem jetzigen Bestrahlungsvorgang die Schicht 47 mit der römischen Ziffer I zuerst bestrahlt wird. Diese Schicht liegt in Strahlrichtung am weitesten hinten und wurde bei dem vorhergehenden Bestrahlungsvorgang (dargestellt in Fig. 2) zuletzt bestrahlt. Anschließend werden die Schichten 47, ausgehend von der in Strahlrichtung hinten liegenden Schicht, sukzessive nach vorne bestrahlt. Zuletzt wird die am weitesten vorne liegende Schicht 47 mit der römischen Ziffer VI bestrahlt. Ebenso wie in Fig. 2 kennzeichnen die römischen Ziffern I bis VI innerhalb der Schichten die Reihenfolge der Bestrahlung der Schichten.

Die Energie des Partikelstrahls muss somit bei einer Bestrahlung von Schicht 47 zu Schicht 47 und bei einer Bestrahlung von Bestrahlungsvorgang (dargestellt anhand von Fig. 2) zu Bestrahlungsvorgang (dargestellt anhand von Fig. 3) lediglich geringfügig geändert werden, so dass insbesondere supraleitende Komponenten des Beschleunigers und/oder des Hochenergiestrahltransportsystems nur geringfügig an die geänderte Energie des Partikelstrahls angepasst werden müssen. Hierdurch treten Belastungen für Komponenten, insbesondere AC-Verluste, in lediglich geringem Umfang auf.

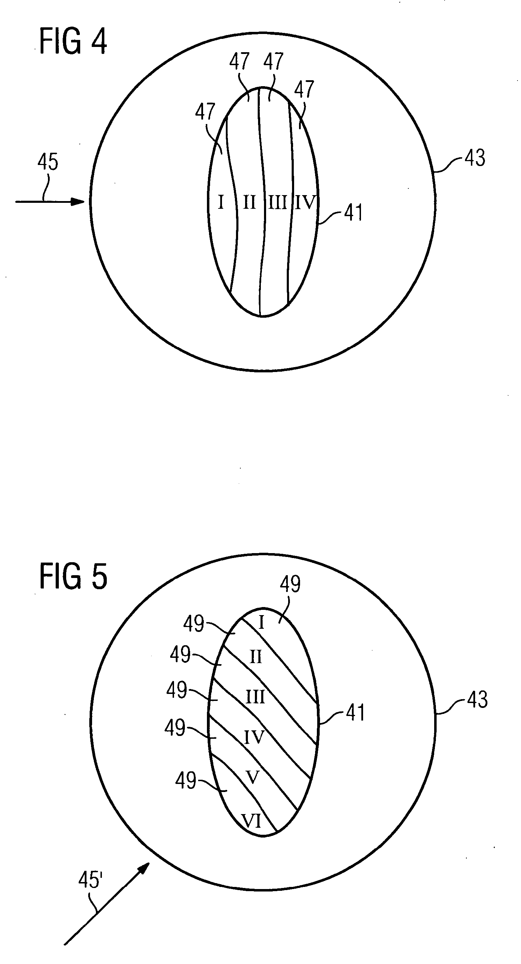

Bei dem in Fig. 2 und Fig. 3 gezeigten Beispiel werden jeweils dieselben Schichten 47 bestrahlt. Dies tritt beispielsweise auf, wenn dasselbe Zielvolumen 41 mehrfach schichtweise abgescannt werden soll. Bei dem in Fig. 4 und Fig. 5 gezeigten Beispiel hingegen wird ein Zielvolumen 41 bei einem Bestrahlungsvorgang von einer Richtung bestrahlt (dargestellt durch den Pfeil 45) und bei dem nächsten Bestrahlungsvorgang von einer anderen Richtung (dargestellt durch den geänderten Pfeil 45'). Da das hier gezeigte Zielvolumen 41 länglich geformt ist, sind zur Bestrahlung des Zielvolumens 41 in einer Richtung vier Schichten 47 notwendig (Fig. 4), während zur Bestrahlung des Zielvolumens in der anderen Richtung sechs weitere Schichten 49 notwendig sind (Fig. 5).

Auch hier wird die Reihenfolge der Schichten 47 bzw. 49 von Bestrahlungsvorgang zu Bestrahlungsvorgang variiert. Bei dem anhand von Fig. 4 dargestellten Bestrahlungsvorgang werden die Schichten 47 von vorne nach hinten bestrahlt - bezogen auf die Strahlrichtung. In Fig. 4 ist dies durch die römischen Ziffern I bis IV in den Schichten angedeutet. In Fig. 5 hingegen wird die Reihenfolge der weiteren Schichten 49 umgedreht, so dass die weiteren Schichten 49 von hinten nach vorn bestrahlt werden. Auch hier stehen die römischen Ziffern I bis VI in den weiteren Schichten 49 für die Reihenfolge der Bestrahlung der weiteren Schichten 49. Die Energie des Partikelstrahls muss bei dem Übergang von einem Bestrahlungsvorgang (dargestellt anhand von Fig. 4) zu dem anderen Bestrahlungsvorgang (dargestellt anhand von Fig. 5) lediglich in geringer Weise angepasst werden. Diese Anpassung ist weitaus geringer als eine Anpassung, in die nötig wäre, wenn die Reihenfolge der Bestrahlung der Schichten 47 bzw. 49 bezogen auf die Strahlrichtung von Bestrahlungsvorgang zu Bestrahlungsvorgang belassen würde.

Die anhand von Fig. 2 bis Fig. 5 erläuterten Bestrahlungsvorgänge können auch variiert werden. So ist es beispielsweise nicht zwingend notwendig, dass bei einem Bestrahlungsvorgang stets alle Schichten des Zielvolumens bestrahlt werden. Beispielsweise kann bei einem Bestrahlungsvorgang jede zweite Schicht bestrahlt werden und in dem darauf folgenden Bestrahlungsvorgang die noch nicht bestrahlten Schichten, diesmal allerdings in umgekehrter Reihenfolge.

Bei einem Übergang von einem Bestrahlungsvorgang zu dem nächsten muss ebenfalls nicht dieselbe Schicht bestrahlt werden, wie es anhand von Fig. 2 und Fig. 3 erläutert wurde; es können beispielsweise auch benachbarte Schichten bestrahlt werden. Auch in diesem Fall findet nur eine lediglich geringe Variation der Energie des Partikelstrahls statt.

## Patentansprüche

1. Partikeltherapieanlage, umfassend
- eine Partikelquelle zur Erzeugung von Partikeln,
- einen Beschleuniger zur Beschleunigung der Partikel und zur Bereitstellung eines hochenergetischen Partikelstrahls,
- eine Hochenergiestrahltransportsystem zur Führung eines aus den beschleunigten Partikeln geformten Partikelstrahls in einen Bestrahlungsraum,
- eine Energieeinstellvorrichtung, mit der eine Energie des Partikelstrahls einstellbar ist, und
- eine Steuervorrichtung zur Steuerung eines Bestrahlungsvorgangs bei der Partikeltherapieanlage, mit der die Energieeinstellvorrichtung steuerbar ist und die derart ausgebildet ist, dass ein Zielvolumen durch zumindest zwei Bestrahlungsvorgänge bestrahlt wird, wobei ein Bestrahlungsvorgang ein Teil einer Bestrahlungssitzung ist, bei dem zumindest ein Teil der räumlich in Strahlrichtung hintereinander angeordneten Schichten des Zielvolumens sukzessive nacheinander bestrahlt wird,
wobei bei jedem Bestrahlungsvorgang eine Energie eines Partikelstrahls derart variiert wird, dass das Zielvolumen schichtweise in Schichten, die in Strahlrichtung räumlich hintereinander angeordnet sind, bestrahlt wird, und
wobei eine Reihenfolge, in der die Schichten des Zielvolumens bei einem der Bestrahlungsvorgänge bestrahlt werden, - bezogen auf eine Einfallsrichtung des Partikelstrahls - von Bestrahlungsvorgang zu Bestrahlungsvorgang in alternierender Richtung durchlaufen wird,
wobei der Beschleuniger und/oder das Hochenergiestrahltransportsystem zumindest einen Magneten, insbesondere einen supraleitenden Magneten, umfassen,
**dadurch gekennzeichnet, dass**
mit der Energieeinstellvorrichtung die Energie des Partikelstrahls in Strahlrichtung vor dem Magneten einstellbar ist, und wobei ein Magnetfeld, das von dem zumindest einen Magneten erzeugt wird, auf die Energie des Partikelstrahls abgestimmt ist.

2. Partikeltherapieanlage nach Anspruch 1, wobei die Steuerungsvorrichtung derart ausgebildet ist, dass die Reihenfolge, in der die Schichten bei einem Bestrahlungsvorgang bestrahlt werden, von Bestrahlungsvorgang zu Bestrahlungsvorgang derart geändert wird, dass beim Übergang von Bestrahlungsvorgang zu Bestrahlungsvorgang eine gleiche Schicht oder benachbarte Schichten bestrahlt werden.

3. Partikeltherapieanlage nach einem der Ansprüche 1 bis 2, wobei die Steuerungsvorrichtung derart ausgebildet ist, dass bei den zumindest zwei Bestrahlungsvorgängen die gleichen Schichten des Zielvolumens bestrahlt werden.

4. Partikeltherapieanlage nach einem der Ansprüche 1 bis 3, wobei die Steuerungsvorrichtung derart ausgebildet ist, dass bei den zumindest zwei Bestrahlungsvorgängen unterschiedliche Schichten des Zielvolumens bestrahlt werden.

5. Partikeltherapieanlage nach einem der Ansprüche 1 bis 4, wobei die Steuerungsvorrichtung derart ausgebildet ist, dass bei den zumindest zwei Bestrahlungsvorgängen eine Einfallsrichtung des Partikelstrahls von Bestrahlungsvorgang zu Bestrahlungsvorgang gleich bleibt.

6. Partikeltherapieanlage nach einem der Ansprüche 1 bis 4, wobei die Steuerungsvorrichtung derart ausgebildet ist, dass bei den zumindest zwei Bestrahlungsvorgängen eine Einfallsrichtung des Partikelstrahls von Bestrahlungsvorgang zu Bestrahlungsvorgang variiert wird.

7. Verfahren zum Steuern der Energie eines Partikelstrahls während einer Bestrahlung eines Zielvolumens, wobei die Bestrahlung eines nicht-lebenden Körpers, Materials und/oder Phantoms durchgeführt wird,
wobei bei der Bestrahlung ein Zielvolumen durch zumindest zwei Bestrahlungsvorgänge bestrahlt wird, wobei ein Bestrahlungsvorgang ein Teil einer Bestrahlungssitzung ist, bei dem zumindest ein Teil der räumlich hintereinander angeordneten Schichten des Zielvolumens sukzessive nacheinander bestrahlt wird,
und
wobei eine Energie eines Partikelstrahls derart variiert wird,
- dass bei jedem Bestrahlungsvorgang das Zielvolumen schichtweise in Schichten, die in Strahlrichtung räumlich hintereinander angeordnet sind, bestrahlt wird, und
- dass eine Reihenfolge, in der die Schichten des Zielvolumens bei einem der Bestrahlungsvorgänge bestrahlt werden, - bezogen auf eine Einfallsrichtung des Partikelstrahls - von Bestrahlungsvorgang zu Bestrahlungsvorgang in alternierender Richtung durchlaufen wird,
**dadurch gekennzeichnet, dass**
die Energie des Partikelstrahls eingestellt wird, bevor der Partikelstrahl durch einen Magneten, insbesondere einen supraleitenden Magneten, eines Beschleunigers oder eines Hochenergiestrahltransportsystems geführt wird.

8. Verfahren nach Anspruch 7,
wobei die Reihenfolge, in der die Schichten des Zielvolumens bei einem der Bestrahlungsvorgänge bestrahlt werden, derart variiert wird, dass beim Übergang von Bestrahlungsvorgang zu Bestrahlungsvorgang eine gleiche Schicht oder benachbarte Schichten bestrahlt werden.

9. Verfahren nach einem der Ansprüche 7 bis 8, wobei bei den zumindest zwei Bestrahlungsvorgängen die gleichen Schichten des Zielvolumens bestrahlt werden.

10. Verfahren nach einem der Ansprüche 7 bis 9, wobei bei den zumindest zwei Bestrahlungsvorgängen unterschiedliche Schichten des Zielvolumens bestrahlt werden.

11. Verfahren nach einem der Ansprüche 7 bis 10, wobei bei den zumindest zwei Bestrahlungsvorgängen eine Einfallsrichtung des Partikelstrahls von Bestrahlungsvorgang zu Bestrahlungsvorgang gleich bleibt.

12. Verfahren nach einem der Ansprüche 7 bis 10, wobei bei den zumindest zwei Bestrahlungsvorgängen eine Einfallsrichtung des Partikelstrahls von Bestrahlungsvorgang zu Bestrahlungsvorgang variiert wird.

13. Partikeltherapieanlage, umfassend
- eine Partikelquelle zur Erzeugung von Partikeln,
- einen Beschleuniger zur Beschleunigung der Partikel und zur Bereitstellung eines hochenergetischen Partikelstrahls,
- eine Hochenergiestrahltransportsystem zur Führung eines aus den beschleunigten Partikeln geformten Partikelstrahls in einen Bestrahlungsraum,
- eine Energieeinstellvorrichtung, mit der eine Energie des Partikelstrahls einstellbar ist, und
- eine Steuervorrichtung zur Steuerung eines Bestrahlungsvorgangs bei der Partikeltherapieanlage, mit der die Energieeinstellvorrichtung steuerbar ist und die derart ausgebildet ist, dass ein Zielvolumen durch zumindest zwei Bestrahlungsvorgänge bestrahlt wird,
wobei bei jedem Bestrahlungsvorgang eine Energie eines Partikelstrahls derart variiert wird, dass das Zielvolumen schichtweise in Schichten, die in Strahlrichtung räumlich hintereinander angeordnet sind, bestrahlt wird, und
wobei eine Reihenfolge, in der die Schichten des Zielvolumens bei einem der Bestrahlungsvorgänge bestrahlt werden, - bezogen auf eine Einfallsrichtung des Partikelstrahls - von Bestrahlungsvorgang zu Bestrahlungsvorgang in alternierender Richtung durchlaufen wird, wobei bei einem Bestrahlungsvorgang jede zweite Schicht bestrahlt wird und in dem darauf folgenden Bestrahlungsvorgang die noch nicht bestrahlten Schichten in umgekehrter Reihenfolge bestrahlt werden,
wobei der Beschleuniger und/oder das Hochenergiestrahltransportsystem zumindest einen Magneten, insbesondere einen supraleitenden Magneten, umfassen,
**dadurch gekennzeichnet, dass**
mit der Energieeinstellvorrichtung die Energie des Partikelstrahls in Strahlrichtung vor dem Magneten einstellbar ist, und wobei ein Magnetfeld, das von dem zumindest einen Magneten erzeugt wird, auf die Energie des Partikelstrahls abgestimmt ist.

14. Verfahren zum Steuern der Energie eines Partikelstrahls während einer Bestrahlung eines Zielvolumens, wobei die Bestrahlung eines nicht-lebenden Körpers, Materials und/oder Phantoms durchgeführt wird,
wobei bei der Bestrahlung ein Zielvolumen durch zumindest zwei Bestrahlungsvorgänge bestrahlt wird,
und
wobei eine Energie eines Partikelstrahls derart variiert wird,
- dass bei jedem Bestrahlungsvorgang das Zielvolumen schichtweise in Schichten, die in Strahlrichtung räumlich hintereinander angeordnet sind, bestrahlt wird, und
- dass eine Reihenfolge, in der die Schichten des Zielvolumens bei einem der Bestrahlungsvorgänge bestrahlt werden, - bezogen auf eine Einfallsrichtung des Partikelstrahls - von Bestrahlungsvorgang zu Bestrahlungsvorgang in alternierender Richtung durchlaufen wird, wobei bei einem Bestrahlungsvorgang jede zweite Schicht bestrahlt wird und in dem darauf folgenden Bestrahlungsvorgang die noch nicht bestrahlten Schichten in umgekehrter Reihenfolge bestrahlt werden,
**dadurch gekennzeichnet, dass**
die Energie des Partikelstrahls eingestellt wird, bevor der Partikelstrahl durch einen Magneten, insbesondere einen supraleitenden Magneten, eines Beschleunigers oder eines Hochenergiestrahltransportsystems geführt wird.

## Claims

1. Particle therapy unit, comprising
- a particle source for the generation of particles,
- an accelerator to accelerate the particles and to provide a high-energy particle beam,
- a high-energy beam transport system to direct a particle beam formed of the accelerated particles into an irradiation chamber,
- an energy adjusting device with which the energy of the particle beam can be adjusted, and
- a control device to control an irradiation procedure in the particle therapy unit, with which the energy adjusting device can be controlled and is embodied such that a target volume is irradiated by at least two irradiation procedures, with an irradiation procedure being part of an irradiation session, in which at least one part of the layers of the target volume, which are arranged spatially one behind the other in the beam direction, is irradiated successively one after the other,
with, during each irradiation procedure, an energy of a particle beam being varied such that the target volume is irradiated layer by layer in layers, said layers being arranged spatially one behind the other in the beam direction and with a sequence, in which the layers of the target volume are irradiated during one of the irradiation procedures, relative to a direction of incidence of the particle beam, being passed through in an alternating direction from irradiation procedure to irradiation procedure,
with the accelerator and/or the high energy beam transport system including at least one magnet, in particular a superconducting magnet,
**characterised in that**
with the energy adjusting device, the energy of the particle beam can be adjusted in the direction of the beam upstream of the magnet, and wherein a magnetic field that is generated by the at least one magnet is synchronised with the energy of the particle beam.

2. Particle therapy unit according to claim 1, with the control device being embodied such that the sequence, in which the layers are irradiated during an irradiation procedure, is changed from irradiation procedure to irradiation procedure such that in the transition from irradiation procedure to irradiation procedure, an identical layer or adjacent layers is/are irradiated.

3. Particle therapy unit according to one of claims 1 to 2, with the control device being embodied such that with the at least two irradiation procedures, the same layers of the target volume are irradiated.

4. Particle therapy unit according to one of claims 1 to 3, with the control device being embodied such that with the at least two irradiation procedures, different layers of the target volume are irradiated.

5. Particle therapy unit according to one of claims 1 to 4, with the control device being embodied such that with the at least two irradiation procedures, a direction of incidence of the particle beam remains identical from irradiation procedure to irradiation procedure.

6. Particle therapy unit according to one of claims 1 to 4, with the control device being embodied such that with the at least two irradiation procedures, a direction of incidence of the particle beam is varied from irradiation procedure to irradiation procedure.

7. Method for controlling the energy of a particle beam during an irradiation of a target volume, with the irradiation of a non-living body, material and/or phantom being implemented,
with, during the irradiation, a target volume being irradiated by at least two irradiation procedures, with an irradiation procedure being part of an irradiation session, in which at least one part of the layers of the target volume, which are arranged spatially one behind the other, is successively irradiated one after the other,
and
with an energy of a particle beam being varied such that
- with each irradiation procedure, the target volume is irradiated layer by layer in layers, which are arranged spatially one behind the other in the beam direction, and
- that a sequence, in which the layers of the target volume are irradiated during one of the irradiation procedures, relative to a direction of incidence of the particle beam, is passed through in an alternating direction from irradiation procedure to irradiation procedure,
**characterised in that**
the energy of the particle beam is adjusted, before the particle beam is guided by a magnet, in particular a superconducting magnet, of an accelerator or a high energy beam transport system.

8. Method according to claim 7,
with the sequence, in which the layers of the target volume are irradiated during one of the irradiation procedures, being varied such that in the transition from irradiation procedure to irradiation procedure, an identical layer or adjacent layers is/are irradiated.

9. Method according to one of claims 7 to 8, with the same layers of the target volume being irradiated during the at least two irradiation procedures.

10. Method according to one of claims 7 to 9, with different layers of the target volume being irradiated during the at least two irradiation procedures.

11. Method according to one of claims 7 to 10, with a direction of incidence of the particle beam remaining identical from irradiation procedure to irradiation procedure during the at least two irradiation procedures.

12. Method according to one of claims 7 to 10, with a direction of incidence of the particle beam being varied from irradiation procedure to irradiation procedure during the at least two irradiation procedures.

13. Particle therapy unit, comprising
- a particle source for the generation of particles,
- an accelerator to accelerate the particles and to provide a high-energy particle beam,
- a high-energy beam transport system to direct a particle beam formed of the accelerated particles into an irradiation chamber,
- an energy adjusting device with which the energy of the particle beam can be adjusted, and
- a control device to control an irradiation procedure in the particle therapy unit, with which the energy adjusting device can be controlled and is embodied such that a target volume is irradiated by at least two irradiation procedures,
with, during each irradiation procedure, an energy of a particle beam being varied such that the target volume is irradiated layer by layer in layers, said layers being arranged spatially one behind the other in the beam direction and with a sequence, in which the layers of the target volume are irradiated during one of the irradiation procedures, relative to a direction of incidence of the particle beam, being passed through in an alternating direction from irradiation procedure to irradiation procedure,
with each second layer being irradiated during an irradiation procedure and in the following irradiation procedure the as yet unirradiated layers being irradiated in the reverse sequence,
with the accelerator and/or the high energy beam transport system including at least one magnet, in particular a superconducting magnet,
**characterised in that**
with the energy adjusting device the energy of the particle beam can be adjusted using the energy adjusting device in the direction of the beam upstream of the magnet, and wherein a magnetic field that is generated by the at least one magnet can be synchronised with the energy of the particle beam.

14. Method for controlling the energy of a particle beam during an irradiation of a target volume, with the irradiation of a non-living body, material and/or phantom being implemented,
with a target volume being irradiated by at least two irradiation procedures during the irradiation
and
with an energy of a particle beam being varied such that
- with each irradiation procedure, the target volume is irradiated layer by layer in layers which are arranged spatially one behind the other in the beam direction and
- that a sequence, in which the layers of the target volume are irradiated during one of the irradiation procedures, relative to a direction of incidence of the particle beam, is passed through in an alternating manner from irradiation procedure to irradiation procedure, with each second layer being irradiated during an irradiation procedure and in the subsequent irradiation procedure, the as yet unirradiated layers being irradiated in reverse sequence,
**characterised in that**
the energy of the particle beam is adjusted before the particle beam is directed by a magnet, in particular a superconducting magnet, of an accelerator or a high energy beam transport system.

## Revendications

1. Installation de thérapie à particules, comprenant
- une source de particules pour produire des particules,
- un accélérateur pour accélérer les particules et pour fournir un faisceau de particules de haute énergie,
- un système de transport de faisceau de haute énergie pour guider un faisceau de particules formé des particules accélérées dans une chambre d'irradiation,
- un dispositif de réglage de l'énergie au moyen duquel on peut régler une énergie du faisceau de particules et
- un dispositif de commande pour commander un processus d'irradiation dans l'installation de thérapie à particules, grâce auquel le dispositif de réglage de l'énergie peut être commandé et qui est exécuté de manière à ce qu'un volume cible soit irradié par au moins deux processus d'irradiation, un processus d'irradiation représentant une partie d'une séance d'irradiation au cour de laquelle au moins une partie des couches du volume cible situées les unes derrière les autres dans l'espace dans la direction du faisceau sont successivement irradiées les unes après les autre,
une énergie d'un faisceau de particules étant modifiée lors de chaque processus d'irradiation de manière à ce que le volume cible soit irradié couche par couche dans des couches situées les unes derrière les autres dans l'espace dans la direction du faisceau et la séquence selon laquelle les couches du volume cible sont irradiées lors de l'un des processus d'irradiation étant parcourue en changeant de sens d'un processus d'irradiation à l'autre - si l'on se réfère à une direction d'incidence du faisceau de particules -,
l'accélérateur et/ou le système de transport de faisceau de haute énergie comprenant au moins un aimant, notamment un aimant supraconducteur, **caractérisé en ce que**
le dispositif de réglage de l'énergie permet de régler l'énergie du faisceau de particules avant l'aimant dans la direction du faisceau et un champ magnétique qui est produit par l'au moins un aimant étant adapté à l'énergie du faisceau de particules.

2. Installation de thérapie à particules selon la revendication 1, le dispositif de commande étant exécuté de manière à ce que la séquence selon laquelle les couches sont irradiées lors d'un processus d'irradiation soit modifié d'un processus d'irradiation à l'autre de façon que, lors du passage d'un processus d'irradiation à l'autre, une même couche ou des couches voisines soi(en)t irradiée(s).

3. Installation de thérapie à particules selon l'une des revendications 1 à 2, le dispositif de commande étant exécuté de manière à ce que les mêmes couches du volume cible soit irradiées lors des au moins deux processus d'irradiation.

4. Installation de thérapie à particules selon l'une des revendications 1 à 3, le dispositif de commande étant exécuté de manière à ce que des couches différentes du volume cible soit irradiées lors des au moins deux processus d'irradiation.

5. Installation de thérapie à particules selon l'une des revendications 1 à 4, le dispositif de commande étant exécuté de manière à ce qu'une direction d'incidence du faisceau de particules demeure constante d'un processus d'irradiation à l'autre lors des au moins deux processus d'irradiation.

6. Installation de thérapie à particules selon l'une des revendications 1 à 4, le dispositif de commande étant exécuté de manière à ce qu'une direction d'incidence du faisceau de particules varie d'un processus d'irradiation à l'autre lors des au moins deux processus d'irradiation.

7. Procédé pour commander l'énergie d'un faisceau de particules pendant une irradiation d'un volume cible, l'irradiation d'un corps non vivant, d'un matériau et/ou d'un fantôme étant réalisée,
un volume cible étant irradié par au moins deux processus d'irradiation lors de l'irradiation, un processus d'irradiation représentant une partie d'une séance d'irradiation au cours de laquelle au moins une partie des couches du volume cible situées les unes derrière les autres dans l'espace sont successivement irradiées les unes après les autre,
et
une énergie d'un faisceau de particules étant modifiée de manière
- à ce que, lors de chaque processus d'irradiation, le volume cible soit irradié couche par couche dans des couches situées les unes derrière les autres dans l'espace dans la direction du faisceau et
- à ce que la séquence selon laquelle les couches du volume cible sont irradiées lors de l'un des processus d'irradiation est parcourue en changeant de sens d'un processus d'irradiation à l'autre - si l'on se réfère à une direction d'incidence du faisceau de particules -,
**caractérisé en ce que**
l'énergie du faisceau de particules est réglée avant que le faisceau de particules soit guidé par un aimant, notamment un aimant supraconducteur, d'un accélérateur ou d'un système de transport de faisceau de haute énergie.

8. Procédé selon la revendication 7,
la séquence selon laquelle les couches du volume cible sont irradiées lors de l'un des processus d'irradiation étant modifiée de manière à ce que, lors du passage d'un processus d'irradiation à l'autre, une même couche ou des couches voisines soi(en)t irradiée(s).

9. Procédé selon l'une des revendications 7 à 8, les mêmes couches du volume cible étant irradiées lors des au moins deux processus d'irradiation.

10. Procédé selon l'une des revendications 7 à 9, des couches différentes du volume cible étant irradiées lors des au moins deux processus d'irradiation.

11. Procédé selon l'une des revendications 7 à 10, une direction d'incidence du faisceau de particules demeurant constante d'un processus d'irradiation à l'autre lors des au moins deux processus d'irradiation.

12. Procédé selon l'une des revendications 7 à 10, une direction d'incidence du faisceau de particules étant modifiée d'un processus d'irradiation à l'autre lors des au moins deux processus d'irradiation.

13. Installation de thérapie à particules, comprenant
- une source de particules pour produire des particules,
- un accélérateur pour accélérer les particules et pour fournir un faisceau de particules de haute énergie,
- un système de transport de faisceau de haute énergie pour guider un faisceau de particules formé des particules accélérées dans une chambre d'irradiation,
- un dispositif de réglage de l'énergie au moyen duquel on peut régler une énergie du faisceau de particules et
- un dispositif de commande pour commander un processus d'irradiation dans l'installation de thérapie à particules, grâce auquel le dispositif de réglage de l'énergie peut être commandé et qui est exécuté de manière à ce qu'un volume cible soit irradié par au moins deux processus d'irradiation,
une énergie d'un faisceau de particules étant modifiée lors de chaque processus d'irradiation de manière à ce que le volume cible soit irradié couche par couche dans des couches situées les unes derrière les autres dans l'espace dans la direction du faisceau et
la séquence selon laquelle les couches du volume cible sont irradiées lors de l'un des processus d'irradiation étant parcourue en changeant de sens d'un processus d'irradiation à l'autre - si l'on se réfère à une direction d'incidence du faisceau de particules -, une couche sur deux étant irradiée lors d'un processus d'irradiation et les couches non encore irradiées étant irradiées dans l'ordre inverse lors du processus d'irradiation qui suit,
l'accélérateur et/ou le système de transport de faisceau de haute énergie comprenant au moins un aimant, notamment un aimant supraconducteur, **caractérisé en ce que**
le dispositif de réglage de l'énergie permet de régler l'énergie du faisceau de particules avant l'aimant dans la direction du faisceau et un champ magnétique qui est produit par le au moins un aimant étant adapté à l'énergie du faisceau de particules.

14. Procédé pour commander l'énergie d'un faisceau de particules pendant une irradiation d'un volume cible, l'irradiation d'un corps non vivant, d'un matériau et/ou d'un fantôme étant réalisée,
un volume cible étant irradié par au moins deux processus d'irradiation lors de l'irradiation
et
une énergie d'un faisceau de particules étant modifiée de manière
- à ce que, lors de chaque processus d'irradiation, le volume cible soit irradié couche par couche dans des couches situées les unes derrière les autres dans l'espace dans la direction du faisceau et
- à ce que la séquence selon laquelle les couches du volume cible sont irradiées lors de l'un des processus d'irradiation est parcourue en changeant de sens d'un processus d'irradiation à l'autre - si l'on se réfère à une direction d'incidence du faisceau de particules -, une couche sur deux étant irradiée lors d'un processus d'irradiation et les couches non encore irradiées étant irradiées dans l'ordre inverse lors du processus d'irradiation qui suit,
**caractérisé en ce que**
l'énergie du faisceau de particules est réglée avant que le faisceau de particules soit guidé par un aimant, notamment un aimant supraconducteur, d'un accélérateur ou d'un système de transport de faisceau de haute énergie.
